(19) Europäisches Patentamt
European Patent Office
Office européen des brevets

(11) **EP 4 309 566 A1**

(12) **EUROPEAN PATENT APPLICATION**
published in accordance with Art. 153(4) EPC

(43) Date of publication:
**24.01.2024 Bulletin 2024/04**

(21) Application number: **21931175.0**

(22) Date of filing: **23.09.2021**

(51) International Patent Classification (IPC):
**A61B 5/00** (2006.01)

(52) Cooperative Patent Classification (CPC):
**A61B 5/00**

(86) International application number:
**PCT/CN2021/120024**

(87) International publication number:
**WO 2022/193600 (22.09.2022 Gazette 2022/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.03.2021 CN 202110293071**

(71) Applicant: **Shenzhen Mindray Bio-Medical Electronics Co., Ltd.**
**Shenzhen, Guangdong 518057 (CN)**

(72) Inventors:
• **WAN, Congying**
  **Shenzhen, Guangdong 518057 (CN)**
• **HUANGFU, Yong**
  **Shenzhen, Guangdong 518057 (CN)**
• **LI, Xiang**
  **Shenzhen, Guangdong 518057 (CN)**

(74) Representative: **KIPA AB**
**Drottninggatan 11**
**252 21 Helsingborg (SE)**

(54) **ANESTHETIC DEPTH INDICATION METHOD, DEVICE FOR PROMPTING ANESTHETIC DEPTH, AND ANESTHESIA MACHINE**

(57) An anesthetic depth indication method, a device for prompting an anesthetic depth, and an anesthesia machine. The method comprises: acquiring respective delivery amounts of at least two drugs respectively applied to a target object during an anesthesia process, and obtaining, on the basis of the respective delivery amounts of the at least two drugs, the current anesthetic effect on the target object when the at least two drugs jointly act on the target object, wherein at least one of the at least two drugs is applied to the target object via intravenous infusion. The anesthetic depth of the target object can be indicated in the case that two anesthetic drugs are applied to the target object, so that a doctor can make a more suitable drug administration decision.

FIG. 3

EP 4 309 566 A1

**Description**

<u>TECHNICAL FIELD</u>

The disclosure relates to the technical field of medical devices, and more particularly to a method for indicating a depth of anesthesia, an apparatus for indicating a depth of anesthesia, and anesthesia machine.

<u>BACKGROUND</u>

Minimum alveolar concentration (MAC) refers to a concentration of anesthetic in the alveoli at one atmospheric pressure, when 50% of patients remain immobile during stimulation of skin incision. During inhalation anesthesia, doctor usually determines a depth of anesthesia of a patient by a MAC value corresponding to the depth of anesthesia. When the concentration of anesthetic in the alveoli of the patient reaches 1.3MAC, about 90% of patients can reach a depth of anesthesia that tolerates stimulation of skin incision. When the concentration of anesthetic in the alveoli of the patient reaches 0.4 MAC, the patient can be awakened by calling and shaking. When doctor simultaneously administers anesthetics, such as sedatives (such as propofol) or analgesics (such as opioids), to the patient through an intravenous infusion, there is a synergistic effect between intravenous anesthetic and inhaled anesthetic, resulting in a decrease in MAC concentration. When using vein and inhalation combined anesthesia, the concentration corresponding to MAC is lower than that when using individually inhalation anesthesia. The patient reaches a deeper depth of anesthesia when receiving the same concentration of inhaled anesthetic. However, the mutual promotion and superposition effects of anesthetics are relatively complex, and the effects of different combinations of anesthetic are not the same. It is difficult for doctor to have a clear understanding of the effect of using intravenous anesthetic and inhaled anesthetic simultaneously, and the doctor can only adjust the concentrations of intravenous anesthetic and of inhaled anesthetic, based on experience. If the doctor lacks experience, it is easy to underestimate the synergistic effect of intravenous anesthetic and inhaled anesthetic, resulting in overuse of anesthetic, deeper anesthesia, longer recovery time, and increased postoperative recovery time for patient. This brings huge challenges to the implementation of precise anesthesia and rapid recovery concept.

<u>SUMMARY</u>

This disclosure mainly provides a method for indicating a depth of anesthesia, an apparatus for indicating a depth of anesthesia, and an anesthesia machine , so as to indicate the depth of anesthesia of a target object.

An embodiment provides a method for indicating a depth of anesthesia, including:

acquiring a first administration amount of a first medication and a second administration amount of a second medication, which are administrated to a target object through an intravenous infusion during an anesthesia process;
determining, based on the first administration amount and the second administration amount, a current anesthetic effect on the target object, wherein the current anesthetic effect is a synergistic effect of the first medication and the second medication on the target object; wherein the current anesthetic effect includes at least one of: a current equivalent MAC value; equivalent amount information of the first medication; equivalent amount information of the second medication; equivalent amount information of a medication, which medication is not the first medication or the second medication, but is capable of being administrated to the target object to generate an anesthetic effect; a probability of responding to stimulation; and a probability of not responding to stimulation; and
displaying the current anesthetic effect.

An embodiment provides a method for indicating a depth of anesthesia, including:

acquiring a third administration amount of a third medication, which is administrated to a target object through a respiratory tract during an anesthesia process;
acquiring a fourth administration amount of at least one fourth medication, which is administrated to the target object through an intravenous infusion during the anesthesia process;
determining, based on the third administration amount and the fourth administration amount, a current anesthetic effect on the target object, wherein the current anesthetic effect is a synergistic effect of the third medication and the at least one fourth medication on the target object; wherein the current anesthetic effect includes at least one of: a current equivalent MAC value; equivalent amount information of the third medication; equivalent amount information of the fourth medication; equivalent amount information of a medication, which medication is not the third medication or the fourth medication, but is capable of being administrated to the target object to generate an anesthetic effect; a probability of responding to stimulation; and a probability of not responding to stimulation; and

displaying the current anesthetic effect.

An embodiment provides an apparatus for indicating a depth of anesthesia, including:

a processor, which is configured to acquire respective administration amounts of at least two medications, which are administrated to a target object during an anesthesia process; and configured to obtain, based on the respective administration amounts of the at least two medications, a current anesthetic effect on the target object, wherein the current anesthetic effect is a synergistic effect of the at least two medications on the target object; wherein at least one of the at least two medications is administrated to the target object through an intravenous infusion; and
a human-machine interaction apparatus, which is configured to display the current anesthetic effect;
wherein the current anesthetic effect includes at least one of: a current equivalent MAC value; equivalent amount information of either of the at least two medications; equivalent amount information of a medication, which medication is not among the at least two medications, but is capable of being administrated to the target object to generate an anesthetic effect; a probability of responding to stimulation; and a probability of not responding to stimulation.

An embodiment provides an anesthesia machine, including:

a delivery apparatus of anesthetic, which is configured to deliver to a target object, a gas which is mixed with at least two inhalants;
an anesthesia respiratory apparatus, which is configured to provide a respiratory support to the target object;
a processor, which is configured to acquire respective administration amounts of the at least two inhalants, which are administrated to the target object during an anesthesia process; and configured to obtain, based on the respective administration amounts of the at least two inhalants, a current anesthetic effect on the target object, wherein the current anesthetic effect is a synergistic effect of the at least two inhalants on the target object;
wherein the current anesthetic effect includes at least one of: equivalent amount information of a first inhalant of the at least two inhalants; equivalent amount information of a second inhalant of the at least two inhalants; or equivalent amount information of a medication, which medication is not the first inhalant or the second inhalant, but is capable of being administrated to the target object to generate an anesthetic effect.

An embodiment provides an anesthesia machine, including:

a delivery apparatus for anesthetic, which is configured to deliver to an anesthesia respiratory apparatus, a gas which is mixed with a medication;
a respiratory loop, which is connected with the anesthesia respiratory apparatus and a target object;
the anesthesia respiratory apparatus, which is configured to provide a respiratory support to the target object, through the respiratory loop;
a monitoring system, which is configured to monitor parameter(s) which is(are) related to ventilation;
a human-machine interaction apparatus, which is configured to display visual information; wherein the visual information includes a main interface, wherein the main interface includes a numerical display area which is configured to display value(s) of at least some of the ventilation related parameter(s), and a waveform display area which is configured to display waveform(s) of at least some of the ventilation related parameter(s); and
a processor, which is configured to acquire a third administration amount of a third medication, which medication is administrated to the target object through the respiratory loop during an anesthesia process; configured to acquire a fourth administration amount of at least one fourth medication, which medication is administrated to the target object through an intravenous infusion during the anesthesia process; configured to obtain, based on the third administration amount, a current MAC value of the third medication, which medication individually produces effect on the target object; configured to obtain, based on the third administration amount and the fourth administration amount, a current anesthetic effect on the target object; wherein the current anesthetic effect includes a current equivalent MAC value of the third medication and the fourth medication, which medication synergistically produce effect on the target object, and the current anesthetic effect is configured to indicate a depth of anesthesia of the target object;
wherein the processor is further configured to control the main interface to display, on a same display screen, the current MAC value and the current equivalent MAC value.

An embodiment provides a computer-readable storage medium on which program(s) is(are) stored, wherein the program(s) is(are) capable of being executed by a processor to implement the method described above.
The method for indicating a depth of anesthesia, apparatus for indicating a depth of anesthesia, and anesthesia machine, according to the above embodiments, acquire respective administration amounts of at least two medications, which are administrated to a target object during an anesthesia process; and obtain, based on the respective administration amounts of the at least two medications, a current anesthetic effect on the target object, wherein the current anesthetic effect is a synergistic effect of the at least two medications on the target object; wherein at least one of the at least two medications is administrated to the target object through an intravenous infusion. Accordingly, this disclosure indicates the depth of anesthesia of the target object, when at least two anesthetics are administrated to the target object, which facilitates doctor to make more suitable decision for medication administration.

BRIEF DESCRIPTION OF THE DRAWINGS

FIG. 1 is a structural block diagram of an apparatus for indicating a depth of anesthesia, according to an embodiment provided by this disclosure.
FIG. 2 is a structural block diagram of an apparatus for indicating a depth of anesthesia, according to another embodiment provided by this disclosure.
FIG. 3 is a flowchart of a method for indicating a depth of anesthesia, according to an embodiment provided by this disclosure.
FIG. 4 is a specific flowchart for step 2 of FIG.3, according to an embodiment provided by this disclosure.
FIG. 5 is a flowchart of a method for indicating a depth of anesthesia, according to an embodiment provided by this disclosure.
FIG. 6 is a diagram of an atrioventricular model in a method for indicating a depth of anesthesia, according to an embodiment provided by this disclosure.
FIG. 7 is a diagram of a five-chamber model in a method for indicating a depth of anesthesia, according to an embodiment provided by this disclosure.
FIG. 8 shows a curve for an effect of medication which changes along with a concentration of the medication, in a method for indicating a depth of anesthesia, according to an embodiment provided by this disclosure.
FIG. 9 shows a surface diagram for a comprehensive effect of two mixed medications, in a method for indicating a depth of anesthesia, according to an embodiment provided by this disclosure.
FIG. 10 is a diagram of a main interface, in a method for indicating a depth of anesthesia, according to an embodiment provided by this disclosure.
FIG. 11 is a partially enlarged view of area A in FIG. 10.

DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described so as to make this disclosure better understood. However, one skilled in the art can easily recognize that some of the features can be omitted in different cases, or can be replaced by other elements, materials and methods. In some cases, some operations related to this disclosure are not shown or described in the description, so as to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or characteristics described in the description may be combined in any appropriate manner to form various embodiments. At the same time, the steps or actions in the method description can also be exchanged or adjusted in order in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc. in the description are operable to distinguish different objects, rather than to describe a specific order. In addition, the terms "connection", and "linkage" mentioned in this disclosure include direct and indirect connection (linkage), unless otherwise specified.

This disclosure calculates a synergistic effect of multiple anesthetics which are administered simultaneously and displays to the doctor for reference an anesthetic effect which is equivalent to a synergistic effect, thus allowing the doctor to intuitively determine the current depth of anesthesia of the patient and make more suitable decision for medication administration. The following embodiments calculate the synergistic effect of multiple medications through pharmacokinetic and pharmacodynamic models, but it should be understood that this disclosure is not limited to these embodiments. The anesthetic in this disclosure can be intravenous medication that produces an anesthetic effect on the patient through an intravenous infusion, or inhalant that produces an anesthetic effect on the patient through a respiratory process. In some cases, the anesthetics are also referred to as anesthetic medications.

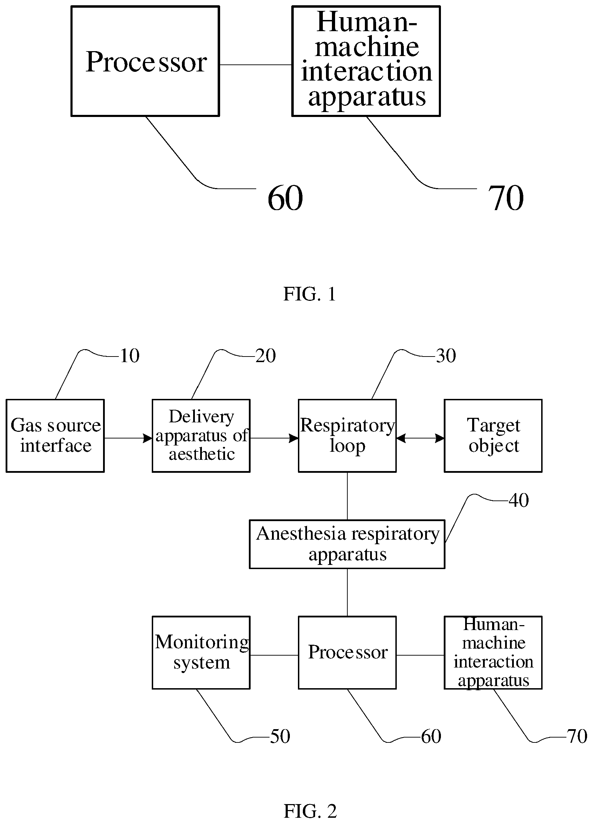

As shown in FIG. 1, an apparatus for indicating a depth of anesthesia, provided by this disclosure, includes a processor 60 and a human-machine interaction apparatus 70.

The human-machine interaction apparatus 70 is used for human-machine interaction, such as outputting visual information and receiving input of user. The human-machine interaction apparatus 70 receives the input of user through an input apparatus, such as a keyboard, operation buttons, a mouse, a trackball, a touchpad, a microphone, etc., or through a touch screen integrated with a display. The human-machine interaction apparatus 70 outputs the visual information through a display, and the type of which is not limited.

The processor 60 is configured to acquire respective administration amounts of at least two medications, which are administrated to a target object during an anesthesia process; and configured to obtain, based on the respective administration amounts of the at least two medications, a current anesthetic effect on the target object, wherein the current anesthetic effect is a synergistic effect of the at least two medications on the target object. The target object is an object being anesthetized, usually a patient. Usually, anesthesia for patient is a continuous process, and the administration of anesthetic is also a continuous process. Therefore, the administration amount can be a concentration, flow rate, partial pressure, dosage, dosage rate, flow rate of intravenous infusion, etc., of the medication.

The depth of anesthesia generally refers to a degree to which general anesthetic suppresses central, circulatory, respiratory functions, as well as stress responses under nociceptive stimulation. The anesthetic effect of this disclosure is a quantitative indicator that can be configured to indicate the depth of anesthesia, quantify the depth of anesthesia, and facilitate measurement by anesthesiologists. Anesthesiologists need to understand the depth of anesthesia of the patient during the anesthesia process, so as to control the administration amount of medication administered. Anesthesiologists are remarkably familiar with various existing anesthetic. Therefore, depths of anesthesia, which correspond to various concentrations and dosages of various anesthetics, probabilities of responding to stimulation for a human, probabilities of not responding to stimulation for a human, MAC values, are well-known to anesthesiologists. Therefore, these can be used as quantitative indicators of the depth of anesthesia, and belong to an anesthetic effect. In this disclosure, the current anesthetic effect on the target object includes at least one of: a current equivalent MAC value; equivalent amount information of either of the at least two medications; equivalent amount information of a medication, which medication is not among the at least two medications, but is capable of being administrated to the target object to generate an anesthetic effect; a probability of responding to stimulation; and a probability of not responding (no response) to stimulation. For the convenience of subsequent description, equivalent amount information of a reference medication is configured to describe the equivalent amount information of a medication, which medication is not among the at least two medications, but is capable of being administrated to the target object to generate an anesthetic effect. Equivalent amount information can include equivalent concentration value or equivalent dosage value, etc. For inhalants, equivalent concentration value is often used. For intravenous infusion medications, equivalent dosage value is often used. Of course, for intravenous infusion medications, an equivalent effect chamber concentration, or a plasma concentration of medication in a plasma chamber can also be used.

After obtaining the current anesthetic effect on the target object, the processor 60 outputs the current anesthetic effect and further utilizes it. For example, the processor 60 outputs the current anesthetic effect to the display of the human-machine interaction apparatus 70 to display the current anesthetic effect, making it easier for doctor to make more suitable decision for medication administration. The display of the human-machine interaction apparatus 70 displays the current anesthetic effect in various ways, such as displaying a numerical value of the current anesthetic effect, or displaying the current anesthetic effect in graphs (such as color blocks) or charts (such as bar charts). User can view an approximate value range of the current anesthetic effect through graphs or charts. For example, different color blocks represents different value intervals of anesthetic effect, the value range of the current anesthetic effect can be determined based on the current color block. For another example, a height of the bar chart presents the value range of the current anesthetic effect. Regardless of the display method, it can reflect the current level of anesthetic effect to a certain extent. The anesthetic effect indicates the depth of anesthesia of the target object. Accordingly, this disclosure indicates the depth of anesthesia of the target object, when at least two anesthetics are administrated to the target object, which facilitates doctor to make more suitable decision for medication administration .

The processor 60 further utilizes the current anesthetic effect. For example, the processor 60 is further configured to acquire a safety range of an anesthetic effect, which is inputted by the user or is preset, and to determine whether the current anesthetic effect is within the safety range of said anesthetic effect, and to output alarm indication information, if the current anesthetic effect is not within the safety range of said anesthetic effect. For example, the processor 60 is further configured to output the alarm indication information to the display, output the alarm indication information to a speaker to emit a corresponding alarm sound, output the alarm indication information to an indicator light to emit a corresponding optical signal, etc., as well as output the alarm indication information to external devices (such as monitors, etc.). Thus, the monitoring of anesthetic effect is achieved, and the safety of anesthesia is improved.

The at least two medications are medications used for anesthesia, which can have multiple combinations. For example, the at least two medications can include at least two inhalants, at least two intravenous infusion medications, and at least one inhalant and at least one intravenous infusion medication. The following provides a detailed explanation of the technical solutions formed by these three-medication combination through multiple embodiments.

Embodiment 1

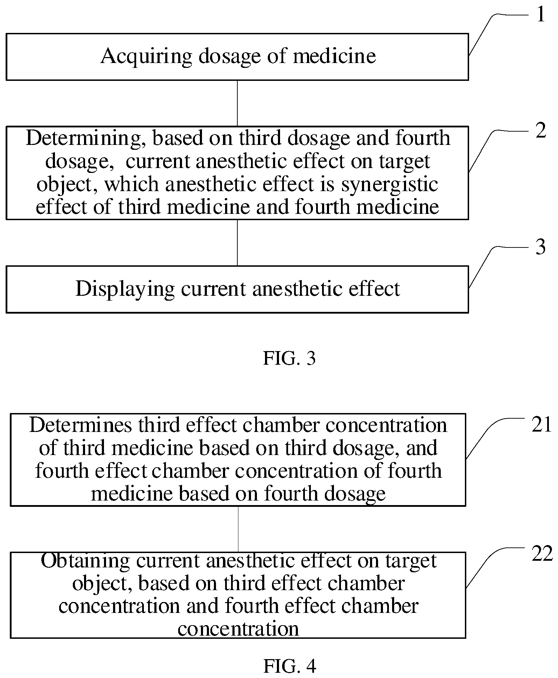

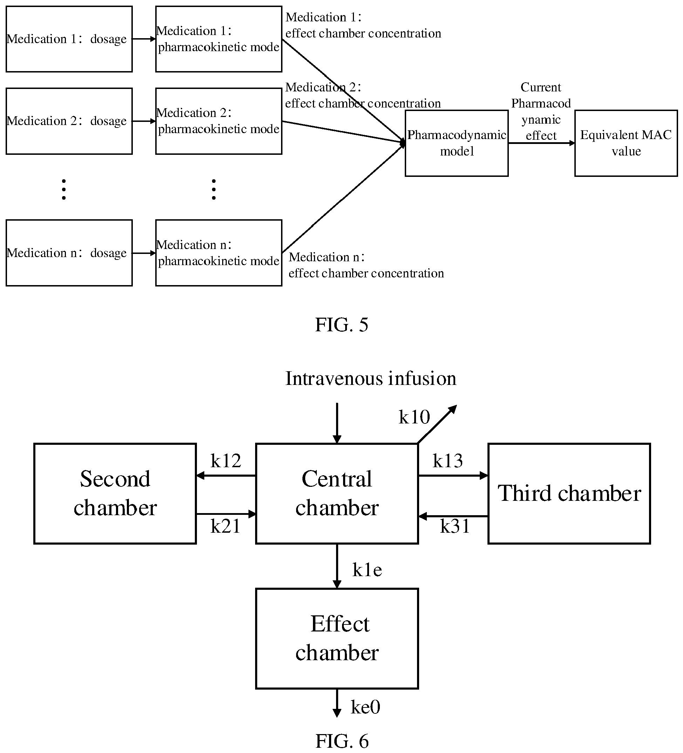

The doctor may use various anesthetics to achieve a desired anesthetic effect during the anesthesia process, such as using propofol and volatile anesthetic inhalant to achieve a sedative effect, and using opioid medication to achieve an analgesic effect. Propofol and opioid medication are administered intravenously, while volatile anesthetic inhalant is administered through respiratory system. In this embodiment, the at least two medications include an inhalant and an intravenous infusion medication. The process of indicating a depth of anesthesia through an apparatus for indicating a depth of anesthesia is shown in FIGS. 3 and 5, and includes following steps.

In step 1, the processor 60 acquires respective administration amounts of each anesthetic during an anesthesia process.

At least, the processor 60 acquires a third administration amount of a third medication, which is administrated to the target object through a respiratory tract during the anesthesia process. For the inhalant, the administration amount can be an effect chamber concentration, which uses the lungs as the effect chamber. In this embodiment, the third administration amount can be a concentration of the third medication in the alveoli, which can be obtained by monitoring the concentration of the third medication at expiratory end of the patient through a sensor. For example, the concentration of the third medication at expiratory end of the patient is used as the concentration of the third medication in the alveoli. The third administration amount of the third medication can also be a flow rate of fresh gas, an output concentration of evaporator, etc. The effect chamber concentration can be calculated through a pharmacokinetic model in subsequent step 21.

The processor 60 acquires a fourth administration amount of at least one fourth medication, which is administrated to the target object through an intravenous infusion during the anesthesia process. There can be two or more types of fourth medication. Wherein, the fourth administration amount can be pill dosage, infusion flow rate, dosage rate, effect chamber concentration or plasma concentration, etc. The effect chamber concentration and plasma concentration are usually difficult to be directly acquired and need to be calculated (referring subsequent step 21). Therefore, the fourth administration amount here is usually pill dosage, infusion flow rate, dosage rate, etc. The processor 60 can acquire the fourth administration amount from an infusion pump.

In step 2, the processor 60 determines a current anesthetic effect on the target object, wherein the current anesthetic effect is a synergistic effect of each anesthetic on the target object, based on the administration amount of each anesthetic. At least, the processor 60 determines, based on the third administration amount and the fourth administration amount, the current anesthetic effect on the target object, wherein the current anesthetic effect is a synergistic effect of the third medication and the fourth medication on the target object. The specific process is shown in FIG. 4, including the following steps.

In step S21, the processor 60 determines an effect chamber concentration of each anesthetic based on the administration amount of each anesthetic. At least, the processor 60 determines a third effect chamber concentration of the third medication based on the third administration amount, and determines a fourth effect chamber concentration of the fourth medication based on the fourth administration amount.

Different medications have different metabolic kinetic characteristics and are suitable for different metabolic kinetic models. For intravenous medications, the commonly used models are the atrioventricular model, with two-chamber and three-chamber models commonly used. Taking the three-chamber model as an example, as shown in FIG. 6, the model includes a central chamber, a second chamber, a third chamber, and an effect chamber. The central chamber represents blood or plasma, the second chamber represents large-scale perfused tissues, the third chamber represents small-scale perfused tissues, and the effect chamber represents an effected part where the medication takes effect, such as brain tissue. Each parameter $k_{ij}$ represents a distribution rate constant. For example, $k_{12}$ represents a distribution rate constant from the central chamber to the second chamber, $k_{21}$ represents a distribution rate constant from the second chamber to the central chamber, $k_{10}$ represents an excretion rate constant from the central chamber, and $k_{e0}$ represents an excretion rate constant from the effect chamber. By calculating a distribution process of medication between different chambers, the medication concentrations in plasma or in the effect chamber of the patient at different time points can be calculated. For different intravenous medications, their pharmacokinetic models may also vary. Based on existing literatures, it is possible to calculate a trend of change(s) in medication concentration in the plasma or effect chamber of the patient for different medications. Therefore, if the fourth administration amount is not the effect chamber concentration, the fourth administration amount can be substituted into a preset pharmacokinetic model of the fourth medication to obtain the fourth effect chamber concentration. If the fourth administration amount is the fourth effect chamber concentration, this step can be omitted and the method directly proceeds to step 22.

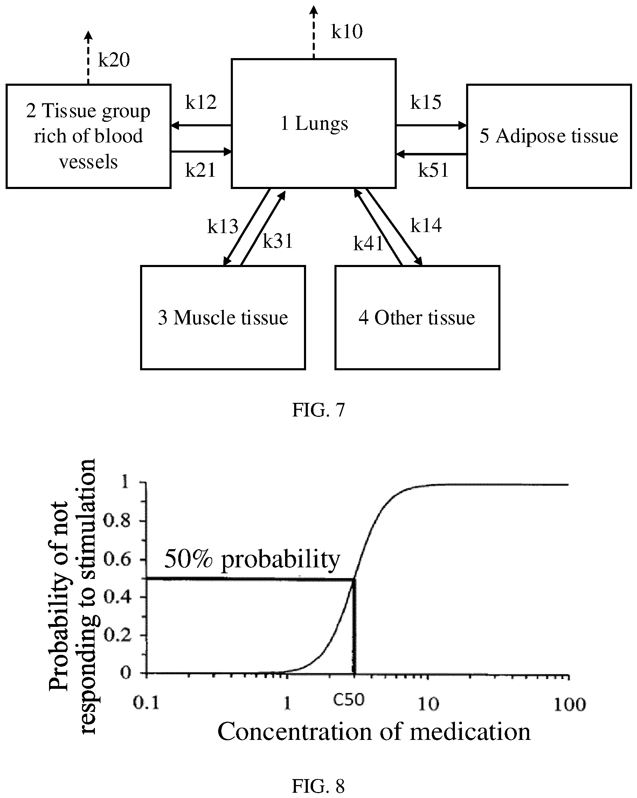

For inhalants, a similar chamber model can also be configured to describe their metabolic kinetics characteristics. Considering their distribution characteristics, a five-chamber model is usually used, as shown in FIG. 7. Wherein, a first chamber presents lungs, a second chamber presents tissue group rich of blood vessels, a third chamber represents muscle tissue, a fifth chamber represents adipose tissue, and a fourth chamber represents other tissues. The inhalant only enters the human body through the first chamber, and there is no inhalant exchange between the chambers. The chambers only exchange the inhalant with the lungs of the first chamber, and then the inhalant is discharged from the human body through the first and second chambers (the function of the liver). Where $k_{ij}$ represents a distribution rate constant of the inhalant between the i-th and j-th chambers, and $k_{i0}$ represents an excretion rate constant of the inhalant. By calculating the distribution process of inhalant between different chambers, the inhalant concentration in the alveoli at different time points can be calculated. This concentration is usually configured to determine the current depth of anesthesia of the patient. Therefore, if the third administration amount is not the effect chamber concentration, the third administration amount can be substituted into a preset pharmacokinetic model of the third medication to obtain the third effect chamber concentration. If the third administration amount is the third effect chamber concentration, this step can be omitted and the method directly proceeds to step 22.

In step 22, the processor 60 obtains the current anesthetic effect on the target object, based on the effect chamber concentration of each anesthetic. At least, the processor 60 obtains the current anesthetic effect on the target object, based on the third effect chamber concentration and the fourth chamber concentration.

Specifically, the processor 60 obtains the current anesthetic effect on the target object, based on the effect chamber concentration of each anesthetic and a preset synergistic pharmacodynamic model corresponding to each anesthetic. At least, the processor 60 inputs the third effect chamber concentration and the fourth chamber concentration into a preset synergistic pharmacodynamic model which corresponds to the third medication and the fourth medication, so as to obtain the current anesthetic effect on the target object. Statistics can be done in advance to possible combinations of various anesthetics, and the corresponding synergistic pharmacodynamic model which corresponds to each combination can be preset in the apparatus for indicating a depth of anesthesia. The current anesthetics, which are administered to the patient, are known, and the processor 60 invokes the corresponding synergistic pharmacodynamic model based on the anesthetics which are administered to the patient, and then obtains the current anesthetic effect on the patient, based on the effect chamber concentrations of these medications. The preset synergistic pharmacodynamic model includes a functional relationship (a pharmacodynamic curve) between each effect chamber concentration and a probability of not responding to stimulation.

For a single medication, its pharmacodynamic model is a model configured to estimate the effect of the medication. The effect E of a single medication can be considered as the probability of not responding to stimulation, which is a function of medication concentration with following formula:

$$E = E_0 + (E_{max} - E_0) \cdot C^\gamma / (C_{50}^\gamma + C^\gamma), \text{Formula 1.}$$

Wherein, E represents an effect generated by the medication, that is, the probability of not responding to stimulation; $E_0$ represents an effect when no medication is administrated; $E_{max}$ represents maximum effect of the medication; $C_{50}$ represents a medication concentration at which there is a 50% probability of not responding to stimulation; $\gamma$ represents a known quantity that reflects a sharp degree of rise of the function; C represents a current medication concentration. A curve of change (pharmacodynamic curve) of the effect E along with a change of the medication concentration C is shown in FIG. 8.

When both the third medication A and the fourth medication B exist, the mixture can be considered as a series of new medications according to different mixing ratios θ. When θ=0, the mixture just includes the fourth medication B, and when θ=1, the mixture just includes the third medication A. By normalizing the concentration with $U=C/C_{50}$, a synergistic pharmacodynamic model formula for the synergistic effect of two medications can be obtained as follows:

$$E = E_0 + (E_{max}(\theta) - E_0) \frac{\left(\frac{U_A + U_B}{U_{50}(\theta)}\right)^{\gamma(\theta)}}{1 + \left(\frac{U_A + U_B}{U_{50}(\theta)}\right)^{\gamma(\theta)}}, \text{Formula 2.}$$

Wherein, $E_0$ represents an effect when no medication is administrated; $E_{max}(\theta)$, $U_{50}(\theta)$ and $r(\theta)$ change when θ changes. $E_{max}(\theta)$ represents maximum synergistic effect of the medication A and the medication B, when the medication A and the medication B has a ratio of θ, wherein $E_{max}(\theta)$ can be obtained based on experience or experiments. For anesthesia, $E_{max}(\theta)$ can be considered as 1. $U_A$ represents a current normalized concentration of medication A, i.e., $U_A = C_A / C_{50}$, wherein $C_A$ represents the current concentration of medication A, and $C_{50}$ represents the concentration of medication A when there is a 50% probability of not responding to stimulation. $U_B$ represents a current normalized concentration of medication B, i.e., $U_B = C_B/C_{50}$, wherein $C_B$ represents the current concentration of medication B, and $C_{50}$ represents the concentration of medication B when there is a 50% probability of not responding to stimulation. $U_{50}(\theta)$ and $r(\theta)$ can be derived from theoretical knowledge or obtained through experiments, and are known quantities. It can be seen that the synergistic pharmacodynamic model of multiple medications can be obtained through the above methods. The above methods are only examples and other methods can be used to obtain the synergistic pharmacodynamic models, which are not elaborated here.

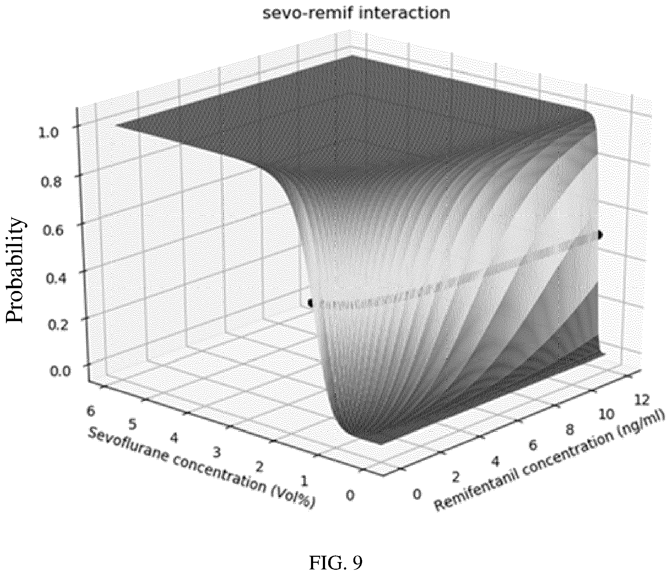

During the anesthesia process, sedative medication and analgesic medication are usually used simultaneously, and the effects of these two medications on the anesthetic effect are mutually synergistically. The infusion of analgesic medication reduces the concentration of sedative medication that achieves the same anesthetic effect. Taking the synergistic pharmacodynamic model of the sedative medication sevoflurane and the analgesic medication remifentanil as an example, a pharmacodynamic surface diagram for the mixture of the two medications is shown in FIG. 9.

The processor 60 normalizes the effect chamber concentration $C_A$ and the effect chamber concentration $C_B$ to obtain $U_A$ and $U_B$. The two are substituted into the synergistic pharmacodynamic model (Formula 2) to obtain a current synergistical effect E, which is the probability of not responding to stimulation, such that the current probability of responding to stimulation can also be obtained. From FIG. 9, it can also be seen that knowing the concentrations of the two medications can yield the synergistic effect E.

If the current anesthetic effect is the current equivalent MAC value, equivalent amount information of the third medication, equivalent amount information of the fourth medication, or equivalent amount information of the reference medication, then the current synergistic effect E needs to be converted into the current anesthetic effect.

For example, the minimum alveolar concentration (MAC) refers to a concentration of anesthetic inhaled into the alveoli at one atmospheric pressure, when 50% of patients or animals lose their escape movement response to harmful stimulation (such as skin incision). The MAC value is a multiple of the minimum alveolar concentration MAC. The MAC value can be considered as the pharmacodynamic effect on the patient for responding to stimulation of skin incision, and doctor has abundant experience of MAC values corresponding to the depths of anesthesia. When the MAC value is 1, the corresponding pharmacodynamic level is that 50% probability of not responding to stimulation of skin incision. When the MAC value is 0.3, the patient is about to wake up, when the MAC value is 1.3, the corresponding pharmacodynamic level is that approximately 90% probability of not responding to stimulation of skin incision. The MAC value is widely known to doctors, such that using it to reflect the depth of anesthesia of patient is both simple and convenient, without increasing the learning cost for doctors. The apparatus for indicating a depth of anesthesia can also include a memory, which calculates and stores the pharmacodynamic curves corresponding to different MAC values in the memory in advance. The processor 60 compares the synergistical effect E of the current two medications after interaction, with the pharmacodynamic curves corresponding to different MAC values when the inhalant is administered individually, and then finds the MAC value corresponding to a same effect. This MAC value is the current equivalent MAC value.

For example, the pharmacodynamic models (pharmacodynamic curves) of the third, fourth, and reference medications are all known, so that the processor 60 can substitute the probability of not responding to stimulation obtained from the synergistic pharmacodynamic model into the pharmacodynamic models of the third, fourth, or reference medications used individually, so as to infer the equivalent amount information of the third, fourth, and reference medications, thereby characterizing the current anesthetic effect.

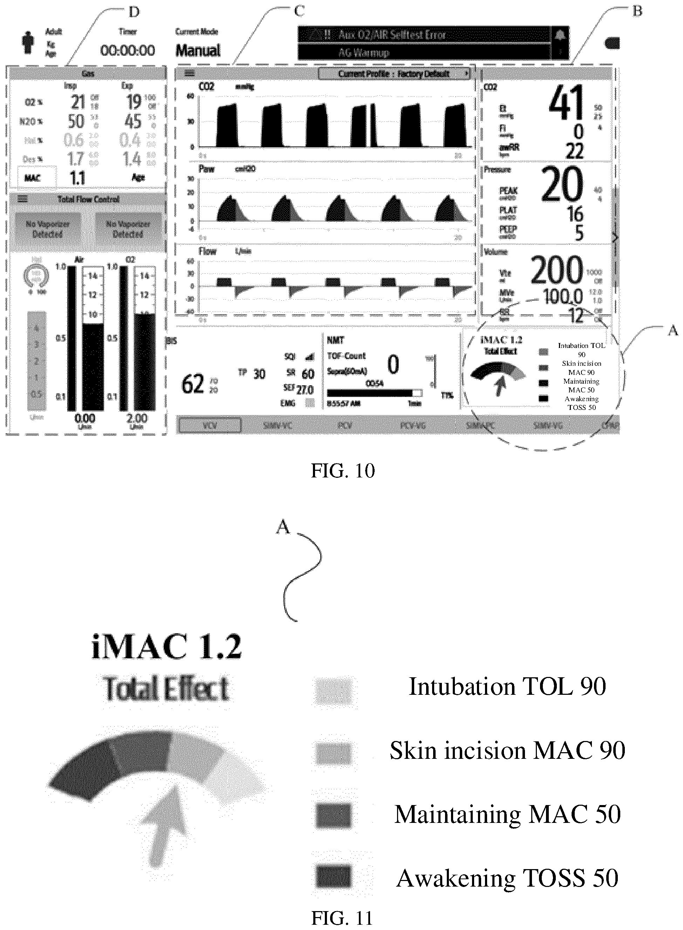

In step 3, the processor 60 displays the current anesthetic effect on the display. For example, the processor 60 displays, in real-time, the current anesthetic effect on the main interface (as shown in FIG. 10). As shown in FIG. 11, when the display shows the current anesthetic effect, the processor 60 can also display operations suitable for the target object under the current anesthetic effect, in a textual or graphic manner through the display, including intubation, skin incision, maintaining or awakening. In this embodiment, the four operations of intubation, skin incision, maintenance, and awakening are displayed in text, and the anesthetic effects corresponding to these four operations are displayed in both graphical (color blocks) and textual (numerical values) formats. The current anesthetic effect is displayed in graphical (color blocks) and textual (numerical values) formats, allowing user to find suitable operations for the target object based on the graphs or text of the current anesthetic effect, which is very intuitive.

Since the processor 60 can calculate the current anesthetic effect based on the administration amount of each medication, it can also further estimate the subsequent anesthetic effect. For example, the processor 60 estimates the third effect chamber concentration of the third medication within a preset future time period based on a preset pharmacokinetic model of the third medication, estimates the fourth effect chamber concentration of the fourth medication within the preset future time period based on a preset pharmacokinetic model of the fourth medication, obtains a trend of change(s) in the anesthetic effect on the target object within the preset future time period, based on the third effect chamber concentration of the third medication within the preset future time period and the fourth effect chamber concentration of the fourth medication within the preset future time period, and then displays, through the display, the trend of change(s) in the anesthetic effect within the preset future time period. The trend of change(s) can be displayed in the form of a graph, such as using a curve of change to show change(s) in the anesthetic effect over time within the preset future time period. The future time period can be preset based on requirements or experiences of the user. For example, a time length of the preset future time period can be longer than a current awakening time of the patient after stopping the current medication administration, which is conducive to calculating the awakening time in the future. By displaying the curve of change in the anesthetic effect of the patient within the preset future time period, anesthesiologists can better understand the subsequent change(s) in the depth of anesthesia of the patient and provide reference for their subsequent medication administration.

The processor 60 can also estimate metabolism time of anesthetic for the target object based on the current anesthetic effect, and display the metabolism time of anesthetic when displaying the current anesthetic effect. Wherein, the metabolism time of anesthetic can include awakening time after stopping the current medication administration. From this, anesthesiologists can not only understand the current depth of anesthesia of the patient, but also receive an indication on how long the patient will wake up after stopping the medication administration, making it easier for anesthesiologists to prepare for subsequent work.

The processor 60 can estimate the metabolism time of anesthetic for the target object based on the current anesthetic effect, by using the following methods.

The processor 60 can acquire an anesthetic effect for awakening, which is inputted by the user or is preset. When the anesthetic effect of patient drops to the anesthetic effect for awakening, it indicates that the patient is about to awaken. The anesthetic effect for awakening can be inputted by the user or preset by the system. Furthermore, the processor 60 estimates the metabolic time from the current anesthetic effect to the anesthetic effect for awakening on the target object, with the metabolic time being the awakening time of the target object after stopping the current medication administration. For example, the processor 60 has obtained the trend of change(s) in the anesthetic effect of the target object within a preset future time period through the above method. From this trend of change(s), the processor 60 can obtain a time point which corresponds to the anesthetic effect for awakening, and obtain the awakening time based on this time point. The awakening time can be the time point in the future when patient wakes up, or a time duration required from the current time point to the time point in the future when patient wakes up, as if it is capable of indicating when the patient wakes up.

The trend of change(s) in the anesthetic effect and the awakening time in the future mentioned above are estimated based on actual administration amount of the medication. The apparatus for indicating a depth of anesthesia provided by this disclosure can also simulate input of administration amount. Specifically, the processor 60 is further configured to provide settings for a future administration amount of medication through a display, and the user can operate the input apparatus to set the future administration amount of each medication. For example, the processor 60 receives respective administration amounts of the at least two medications within a preset future time period, which administration amounts are preset by the user through the input apparatus, such as receiving the third administration amount of the third medication and the fourth administration amount of the fourth medication within the preset future time period preset by the user. Based on the respective administration amounts of the at least two medications within the preset future time period, the processor 60 obtain the trend of change(s) in the anesthetic effect within the preset future time period. For example, based on the third administration amount and the fourth administration amount within the preset future time period, the processor 60 obtains the trend of change(s) in the anesthetic effect within the preset future time period. For example, the processor 60 substitutes the third administration amount within the preset future time period into the preset pharmacokinetic model of the third medication, and estimates the third effect chamber concentration of the third medication within the preset future time period; substitutes the fourth administration amount within the preset future time period into the preset pharmacokinetic model of the fourth medication, and estimates the fourth effect chamber concentration of the fourth medication within the preset future time period; and obtains the trend of change(s) in the anesthetic effect within the preset future time period on the target object, based on the third effect chamber concentration of the third medication within the preset future time period and the fourth effect chamber concentration of the fourth medication within the preset future time period. Then the processor 60 displays the trend of change(s) in the anesthetic effect during the preset time period through the display. Similarly, the trend of change(s) can be a graph including curve of change(s) that reflects a relationship between the anesthetic effect and time. By using the above method, anesthesiologists can input the administration amounts of various medications before anesthesia, and then simulate the trend of change(s) in the anesthetic effect through the apparatus. This provides a reference for anesthesiologists to ultimately determine the administration amount of patient, improving the work efficiency of anesthesiologists and the safety of patient anesthesia.

The processor 60 is further configured to acquire a target anesthetic effect inputted by the user through the input apparatus. Of course, the target anesthetic effect can also be preset by the system, that is, the processor 60 can also obtain a preset target anesthetic effect. When the processor 60 displays the trend of change(s) in the anesthetic effect within the preset future time period through the display, it can also display the target anesthetic effect through the display. This can indicate how long it will take for the patient to achieve the target anesthetic effect. Of course, the processor 60 can also obtain a time length required for the anesthetic effect to reach the target anesthetic effect based on the target anesthetic effect and the trend of change(s) in the anesthetic effect within the preset future time period. When the trend of change(s) in the anesthetic effect within the preset future time period is displayed on the display, this time length is also displayed, so as to give direct indication. The trend of change(s) in the anesthetic effect within the preset future time period can be based on the actual administration amount of medication, or based on the third administration amount of the third medication and the fourth administration amount of the fourth medication within the preset future time period, which administration amounts are set by the user. Both methods are described in the above description.

The processor 60 can also be configured to receive a modification from the user to the administration amount of at least one medication of at least two medications through the input apparatus. For example, the processor 60 receives a modification from the user to the administration amount of the third medication, and/or receives a modification from the user to the administration amount of the fourth medication, and then updates and displays through the display, the trend of change(s) in the anesthetic effect within the preset future time period, based on the modification from the user to the administration amount of at least one medication. In this way, when doctor modifies the administration amount of medication, the trend of change(s) in the anesthetic effect can also be updated at any time, and the indication of anesthetic effect is very convenient and accurate. If the modification is the administration amount of medication which is administered in the future, it can also update the trend of change(s) in the anesthetic effect, making it more convenient for the doctor to simulate the administration amount of medication administered.

This disclosure can not only estimate the anesthetic effect in the further, but also can review the anesthetic effect in the past. The processor 60 associates and saves the current anesthetic effect with a current time point, and after a time period, saves the anesthetic effects during a previous time period. The doctor can output corresponding instructions through the input apparatus. The processor 60 receives an instruction to view a change condition of the anesthetic effect through the input apparatus, and in response to this instruction, generates the trend of change(s) in the anesthetic effect based on the anesthetic effects at different time points during the anesthesia process (a time period before the current time point), and displays the same on the display. In this embodiment, displaying the trend of change(s) is illustrated by displaying a graph including curve of change(s) as an example. Doctor can use the graph including curve of history change(s) of the patient for teaching and for easy traceability of surgical conditions.

When providing historical data of anesthetic effect, the processor 60 can also provide corresponding administration amount of medication for reference of doctor. For example, the processor 60 generates a trend of change(s) (such as a curve of change) in the administration amount of medication based on the administration amounts of medication at different times during the anesthesia process (a time period before the current time point) and displays, on the trend of change(s) in the anesthetic effect (such as a graph including a curve of change(s)), the trend of change(s) in the administration amount of medication. The trend of change(s) in the administration amount of medication and the trend of change(s) in the anesthetic effect share a time coordinate axis. In this way, doctor can not only view the trend of change(s) in the administration amount of medication and the trend of change(s) in the anesthetic effect, on the graph including a curve of change(s) of anesthetic effect, but also view the corresponding relationship between the two, which has reference significance. For example, the processor 60 can receive an instruction to select a time point at the trend of change(s) in the anesthetic effect through the input apparatus, and in response to this instruction, display the administration amount of medication corresponding to the selected time point. This can also reflect the corresponding relationship between the administration amount of medication administered at a certain time point and the anesthetic effect.

The apparatus for indicating a depth of anesthesia can be a medical device, such as an anesthesia machine, a ventilator, a monitor, a central station, and other devices with data processing capabilities. This embodiment takes an anesthesia machine as an example for explanation.

As shown in FIG. 2, an anesthesia machine provided by this disclosure includes a gas source interface 10, a delivery apparatus for anesthetic 20, a respiratory loop 30, an anesthesia respiratory apparatus 40, a monitoring system 50, a processor 60, and a human-machine interaction apparatus 70. The gas source interface 10 is connected with the respiratory loop 30 through the delivery apparatus for anesthetic 20, that is, the three are sequentially connected through a gas pipeline.

The gas source interface 10 is connected with a gas source. The gas source is configured to provide gases required for the anesthesia machine, such as oxygen, air, and laughing gas (nitrous oxide). The gas source can be external, such as various gas cylinders, hospital pipelines, etc., or can be internal, such as a turbine.

The delivery apparatus for anesthetic is configured to mix the gases provided by the gas sources with a third medication (anesthetic inhalant), and deliver the mixed gas to the respiratory loop 30. The delivery apparatus for anesthetic 20 can use an evaporator. A gas mixing pipeline, which consists of connection catheters and various valves, can be connected in series between the gas source interface 10 and the delivery apparatus for anesthetic 20, so as to converge oxygen, air, and laughing gas (nitrous oxide) from interfaces of gas sources and output the mixed gas to the delivery apparatus for anesthetic 20. Some valves can close the corresponding pipelines of oxygen, air, laughing gas and other gases, while others can adjust the quantity of flow of corresponding oxygen, air, laughing gas and other gases. By controlling the various valves, various gas flow rates, mixing ratios, etc., can be set.

The respiratory loop 30 is a gas path connecting the delivery apparatus for anesthetic and the target object (usually the patient), which can recycle the gas exhaled by the patient to save anesthetic and reduce environmental pollution. The respiratory loop 30 can include various connection catheters and accessories, such as nasal plugs, nasal masks, masks, tracheal catheters with airbags at ends, etc. A gas purification apparatus can be installed inside the respiratory loop 30, and configured to remove at least a portion of carbon dioxide exhaled by the patient into the respiratory loop. $CO_2$ absorbent (sodium lime) can be installed inside the gas purification apparatus. By reacting with $CO_2$ absorbent, the goal of removing $CO_2$ is achieved, while generating water and heat through the reaction, which is beneficial for maintaining the temperature and humidity of the gas inhaled by the patient.

The anesthesia respiratory apparatus 40 is configured to control the respiratory loop 30 to periodically deliver the mixed gas to the patient, thereby providing anesthesia respiratory support for the patient. For example, the anesthesia respiratory apparatus 40 may include multiple valves and cards for driving the multiple valves, wherein the multiple valves are installed inside the respiratory loop 30. The cards control multiple valves to periodically deliver the mixed gas to the patient, thereby providing anesthesia respiratory support for the patient.

The monitoring system 50 is configured to monitor ventilation related parameters. The ventilation related parameters can include flow rates of various gases, for example, the monitoring system 50 includes multiple flow sensors, which are configured to monitor the flow rates of various gases (oxygen, air, laughing gas, third medication, etc.). The monitoring system 50 further includes multiple pressure sensors to monitor a pressure in each gas path, including a pressure of carbon dioxide exhaled by the patient, and to monitor a pressure inside a body of the patient, such as airway pressure and esophageal pressure.

The processor 60 is configured to control the delivery apparatus for anesthetic 20, the anesthesia respiratory apparatus 40, the monitoring system 50, and the human-machine interaction apparatus 70, etc., so as to achieve the functions of the anesthesia machine.

During the operation of the anesthesia machine, the gas source provides gas, which is mixed through the gas mixing pipeline for gas composition mixing. Then, the third medication is added and the concentration of the third medication is adjusted through the evaporator to form fresh gas. The fresh gas enters the respiratory loop 30 and is controlled through manual balloon or the anesthesia respiratory apparatus 40 for ventilation, so as to deliver the fresh gas to the patient. The exhaust gas exhaled by the patient passes through the gas purification apparatus, and is either purified or discharged after the purification. During the above process, the anesthesia respiratory apparatus 40 monitors machine states and patient parameters through the monitoring system 50, ensuring patient safety and providing abnormal alarms.

The processor 60 displays a main interface through the display, as shown in FIG. 10. The main interface includes a numerical display area B for displaying values of at least some ventilation related parameters, and a waveform display area C for displaying waveforms of at least some ventilation related parameters.

The processor 60 obtains a current MAC value of the third medication, which individually produces effect on the target object, based on the third administration amount. For example, the processor 60 inputs the third administration amount into the preset pharmacokinetic model of the third medication to obtain the third effect chamber concentration of the third medication, inputs the third effect chamber concentration into the preset pharmacodynamic model of the third medication to obtain the functional relationship between the third effect chamber concentration and the probability of not responding to stimulation, so as to obtain the current MAC value of the third medication, which individually produces effect on the target object, based on this functional relationship. As shown in FIG.10, the current MAC value is 1.1. In this embodiment, the current anesthetic effect includes the current equivalent MAC value of the third medication and the fourth medication which synergistically produce effect on the target object.

The processor 60 controls the display to display the current MAC value and the current equivalent MAC value on the main interface on the same screen. In FIG. 10, the current MAC value is 1.1, and the current equivalent MAC value is 1.2. Specifically, the main interface also includes an indication area for inhalation anesthesia D for displaying parameters related to administration of the third medication. The parameters related to administration of the third medication include, for example, oxygen concentration, nitrous oxide concentration, oxygen flow rate, air flow rate, etc. The processor 60 controls the display to display the current MAC value in the indication area for inhalation anesthesia D, and to display the current equivalent MAC value in an area, which is different from the indication area for inhalation anesthesia D (area A in FIG. 10). Of course, in other embodiments, the current MAC value and the current equivalent MAC value can also be displayed adjacently, making it easier for doctor to understand the increase in the anesthetic effect after intravenous anesthesia.

The MAC value can be considered as the effect on the patient for responding to stimulation of skin incision, and doctors have abundant experience of MAC values corresponding to the depths of anesthesia, so that no learning costs are resulted for doctors. The equivalent MAC value ultimately obtained by this disclosure can be presented in a numerical display, a graphical display, a differentiation display of different color blocks, etc.

It can be seen that the anesthesia machine provided by this disclosure can provide doctors with synergistic effect indications of at least two types of anesthetics without increasing the learning cost of doctors, while patients have both inhaled and intravenous anesthetics, making it easy for doctors to understand the anesthesia situation of the current patient. The anesthetic mentioned in disclosure are various medications used for anesthesia.

Embodiment 2:

In this embodiment, steps of the method for indicating a depth of anesthesia are also shown in FIG. 3. Due to changes in the medications, the third medication is replaced by a first medication, the fourth medication is replaced by a second medication, the third administration amount of the third medication is replaced by a first administration amount of the first medication and the fourth administration amount of the fourth medication is replaced by a second administration amount of the second medication. The method for indicating a depth of anesthesia includes following steps.

In step 1', the processor 60 acquires respective administration amounts of each anesthetic during an anesthesia process. The difference between this embodiment and embodiment 1 is that both the first medication and the second medication in this embodiment are intravenous infusion medications, that is, both the first medication and the second medication are administered intravenously. Therefore, in this embodiment, step 1' specifically involves following steps. The processor 60 acquires a first administration amount of the first medication and a second administration amount of the second medication which are administrated to a target object through an intravenous infusion during the anesthesia process. The first administration amount and the second administration amount can be acquired from an infusion pump, manually inputted by the user, or acquired from other hospital information system through the anesthesia machine.

The first, second, and fourth medications of this disclosure all involve anesthetics which produce effect on the target object through the intravenous infusion. In embodiments 1 and 2, the fourth medication can be the same intravenous infusion medication as the first medication or the second medication, or another intravenous infusion medication different from the first medication and the second medication.

In step 2', the processor 60 determines, based on the first administration amount and the second administration amount, a current anesthetic effect on the target object, wherein the current anesthetic effect is a synergistic effect of the first medication and the second medication on the target object. Similarly, the specific process can be achieved using the method shown in FIG. 4, and just need to replace the third administration amount of the third medication by the first administration amount of the first medication, and to replace the fourth administration amount of the fourth medication by the second administration amount of the second medication. In other words, the difference from embodiment 1 is that the preset pharmacokinetic models used in this embodiment are all atrioventricular models. The other content is the same as step 2 of embodiment 1, and is not elaborated here.

In step 3', the processor 60 displays the current anesthetic effect on the display. The specific process is the same as step 3 of embodiment 1, and is not elaborated here.

In embodiments 1 and 2, at least one of the at least two medications is administrated to the target object through the intravenous infusion. Of course, the at least two medications can also be inhalants, as explained by embodiment 3 below.

Embodiment 3:

In this embodiment, steps of the method for indicating a depth of anesthesia are also shown in FIG. 3. Due to changes in the medications, the third medication is replaced by a first inhalant, the fourth medication is replaced by a second inhalant, the third administration amount of the third medication is replaced by a first administration amount of the first inhalant and the fourth administration amount of the fourth medication is replaced by a second administration amount of the second inhalant. The method for indicating a depth of anesthesia includes following steps.

In step 1", the processor 60 acquires respective administration amounts of each anesthetic during an anesthesia process. The difference between this embodiment and embodiment 1 is that both the medications used in this embodiment are inhalants, that is, both the first and second medications are administered through the respiratory tract. The other content of this embodiment is the same as that of the embodiment 1. Therefore, in this embodiment, step 1 specifically includes following steps. The processor 60 acquires the respective administration amounts of the at least two inhalants which are administrated to the target object during the anesthesia process, such as the first administration amount of the first inhalant and the second administration amount of the second inhalant which are administrated to the target object through the respiratory tract during the anesthesia process.

In step 2", the processor 60 determines a current anesthetic effect on the target object, wherein the current anesthetic effect is a synergistic effect of the at least two inhalants on the target object, based on the respective administration amount of the at least two inhalants. For example, the processor 60 determines a current anesthetic effect on the target object, wherein the current anesthetic effect is a synergistic effect of the at least two inhalants on the target object, based on the first administration amount of the first inhalant and the second administration amount of the second inhalant. Similarly, the specific process can be achieved using the method shown in FIG. 4, and just need to replace the third administration amount of the third medication to the first administration amount of the first inhalant and to replace the fourth administration amount of the fourth medication to the second administration amount of the second inhalant.

In other words, the difference between this embodiment and embodiment 1 is that the preset pharmacokinetic models used in this embodiment are all five-chamber models. The other content is the same as step 2 of embodiment 1, and is not elaborated here.

In step 3", the processor 60 displays the current anesthetic effect on the display. The current anesthetic effect includes equivalent amount information of the first inhalant of the at least two inhalants; equivalent amount information of the second inhalant of the at least two inhalants; equivalent amount information of a medication, which medication is not the first inhalant or the second inhalant, but is capable of being administered to the target object to generate an anesthetic effect. The specific process is the same as step 3 of embodiment 1, and is not elaborated here.

As understood by those skilled in the art, all or part of the functions of various methods in the above implementation methods can be achieved through hardware or computer programs. When all or part of the functions in the above embodiments are implemented through a computer program, the program can be stored in a computer-readable storage medium, which can include read-only memory, random access memory, magnetic disk, optical disk, hard disk, etc. The program is executed by a computer to achieve the above functions. For example, the program can be stored inside a memory of a device and when the program stored inside the memory is executed through a processor, all or part of the above functions can be achieved. In addition, when all or part of the functions in the above embodiments are implemented through computer programs, the program can also be stored in a storage media, such as server, another computer, disk, CD, flash drives or mobile hard drive, and can be downloaded or copied to the memory of a local device, or can update versions of the system of the local device, and when the program in the memory is executed through the processor, all or part of the functions in the above implementation can be achieved.

In addition, as understood by those skilled in the art, the principles of disclosure can be reflected in computer program products on computer readable storage media, which are pre-installed with computer readable program code. Any tangible, non-temporary computer-readable storage medium can be used, including magnetic storage devices (hard drives, floppy disks, etc.), optical storage devices (CD-ROM, DVD, Blu Ray disks, etc.), flash memory, and/or the like. These computer program instructions can be loaded onto general-purpose computers, specialized computers, or other programmable data processing devices to form machines, allowing these instructions executed on computers or other programmable data processing devices to generate devices that implement specified functions. These computer program instructions can also be stored in a computer readable memory, which can instruct a computer or other programmable data processing device to run in a specific way, so that the instructions stored in computer readable memory can form a manufacturing product, including implementation devices to achieve the specified functions. Computer program instructions can also be loaded onto a computer or other programmable data processing device to perform a series of operational steps on the computer or other programmable device to generate a computer implemented process, allowing instructions executed on the computer or other programmable device to provide steps for implementing specified functions.

Although the principles of disclosure have been demonstrated in various embodiments, many modifications of structures, arrangements, proportions, components, materials, and elements that are particularly suitable for specific environments and operational requirements can be used without departing from the principles and scope of this disclosure. The above modifications and other changes or revisions will be included within the scope of disclosure.

The above specific description has been described with reference to various embodiments. However, those skilled in the art recognize that various modifications and changes can be made without departing from the scope of this disclosure. Therefore, the consideration of this disclosure is explanatory rather than restrictive, and all these modifications are included within its scope. Similarly, there are solutions for the advantages, other advantages, and problems of various embodiments as described above. However, the benefits, advantages, solutions to problems, and any solutions that can generate or make them clearer should not be interpreted as critical, necessary, or indispensably. The term "including" and any other variations thereof used in disclosure belong to non-exclusive inclusion, which includes not only these elements but also other elements that are not explicitly listed or do not belong to the process, method, system, article, or device. In addition, the term "coupling" and any other variations thereof used in disclosure refer to physical connection, electrical connection, magnetic connection, optical connection, communication connection, functional connection, and/or any other connection.

Those skilled in the art recognize that many changes can be made to the details of the aforementioned embodiments without departing from the basic principles of this disclosure. Therefore, the scope of this disclosure should be determined according to the following claims.

## Claims

1. A method for indicating a depth of anesthesia, **characterized in that**, comprising:

   acquiring a first administration amount of a first medication and a second administration amount of a second medication, which medications are administered to a target object through an intravenous infusion during an anesthesia process;

determining, based on the first administration amount and the second administration amount, a current anesthetic effect on the target object, wherein the current anesthetic effect is a synergistic effect of the first medication and the second medication on the target object; wherein the current anesthetic effect comprises at least one of: a current equivalent MAC value; equivalent amount information of the first medication; equivalent amount information of the second medication; equivalent amount information of a medication, which medication is not the first medication or the second medication, but is capable of being administrated to the target object to generate an anesthetic effect; a probability of responding to stimulation; and a probability of not responding to stimulation; and

displaying the current anesthetic effect.

2. A method for indicating a depth of anesthesia, **characterized in that**, comprising:

acquiring a third administration amount of a third medication, which medication is administrated to a target object through a respiratory tract during an anesthesia process;

acquiring a fourth administration amount of at least one fourth medication, which medication is administrated to the target object through an intravenous infusion during the anesthesia process;

determining, based on the third administration amount and the fourth administration amount, a current anesthetic effect on the target object, wherein the current anesthetic effect is a synergistic effect of the third medication and the at least one fourth medication on the target object; wherein the current anesthetic effect comprises at least one of: a current equivalent MAC value; equivalent amount information of the third medication; equivalent amount information of the fourth medication; equivalent amount information of a medication, which medication is not the third medication or the fourth medication, but is capable of being administrated to the target object to generate an anesthetic effect; a probability of responding to stimulation; and a probability of not responding to stimulation; and

displaying the current anesthetic effect.

3. The method according to claim 1 or claim 2, **characterized in that**, further comprising:

displaying, in a textual or graphic manner, an operation which is suitable for the target object under the current anesthetic effect, wherein the operation comprises intubation, skin incision, maintaining or awakening.

4. The method according to claim 1 or claim 2, **characterized in that**, further comprising:

estimating metabolism time of anesthetic for the target object according to the current anesthetic effect, and displaying the metabolism time of anesthetic, when the current anesthetic effect is displayed.

5. The method according to claim 4, **characterized in that**, the metabolism time of anesthetic comprises awakening time after stopping current medication administration;

estimating metabolism time of anesthetic for the target object according to the current anesthetic effect, comprises:

acquiring an anesthetic effect for awakening, which is inputted by a user or is preset; and

estimating the metabolism time of anesthetic for the target object from the current anesthetic effect to the anesthetic effect for awakening, wherein the metabolism time of anesthetic for the target object is the awakening time after stopping current medication administration for the target object.

6. The method according to any one of claims 1-5, **characterized in that**, further comprising:

acquiring a safety range of an anesthetic effect, which is inputted by a user or is preset; and

determining whether the current anesthetic effect is within the safety range of said anesthetic effect, and outputting alarm indication information, if the current anesthetic effect is not within the safety range of said anesthetic effect.

7. The method according to any one of claims 1-5, **characterized in that**, further comprising:

receiving an instruction to view a change condition of an anesthetic effect;

generating, in response to said instruction, a graph including a curve for change(s) in said anesthetic effect, according to anesthetic effects at different time points during the anesthesia process; and

displaying the graph including said curve for change(s) in said anesthetic effect.

8. The method according to claim 7, **characterized in that**, further comprising:

generating a curve for change(s) in an administration amount of medication, according to administration amounts of medication at different time points during the anesthesia process; and displaying, in the graph, the curve for change(s) in the administration amount of medication; wherein the curve for change(s) in the administration amount of medication and the curve for change(s) in said anesthetic effect share a common coordinate axis of time; or

receiving an instruction to select a time point in the graph, and displaying, in response to said instruction, an administration amount of medication which corresponds to the selected time point.

9. The method according to any one of claims 1-5, **characterized in that**, determining, based on the first administration amount/the third administration amount and the second administration amount/the fourth administration amount, a current anesthetic effect on the target object, wherein the current anesthetic effect is a synergistic effect of the first administration amount/the third administration amount and the second administration amount/the fourth administration amount on the target object, comprises:

determining a second effective chamber concentration/a fourth effective chamber concentration of the second medication/the fourth medication, according to the second administration amount/the fourth administration amount, when the first administration amount/the third administration amount is a first effective chamber concentration/a third effective chamber concentration of the first medication/the third medication; and obtaining the current anesthetic effect on the target object, according to the first effective chamber concentration/the third effective chamber concentration and the second effective chamber concentration/the fourth effective chamber concentration; or

determining a first effective chamber concentration/a third effective chamber concentration of the first medication/the third medication, according to the first administration amount/the third administration amount; determining a second effective chamber concentration/a fourth effective chamber concentration of the second medication/the fourth medication, according to the second administration amount/the fourth administration amount; and obtaining the current anesthetic effect on the target object, according to the first effective chamber concentration/the third effective chamber concentration and the second effective chamber concentration/the fourth effective chamber concentration.

10. The method according to claim 9, **characterized in that**, further comprising:

estimating the first effective chamber concentration/the third effect chamber concentration of the first medication/the third medication within a preset future time period, based on a preset pharmacokinetic model of the first medication/a preset pharmacokinetic model of the third medication;

estimating the second effective chamber concentration/the fourth effect chamber concentration of the second medication/the fourth medication within the preset future time period, based on a preset pharmacokinetic model of the second medication/a preset pharmacokinetic model of the fourth medication; and

obtaining a trend of change(s) in an anesthetic effect on the target object within the preset future time period, based on the first effective chamber concentration/the third effect chamber concentration of the first medication/the third medication within the preset future time period, and the second effective chamber concentration/the fourth effect chamber concentration of the second medication/the fourth medication within the preset future time period; and

displaying the trend of change(s) in said anesthetic effect within the preset future time period.

11. The method according to claim 9, **characterized in that**, obtaining the current anesthetic effect on the target object, according to the first effective chamber concentration/the third effective chamber concentration and the second effective chamber concentration/the fourth effective chamber concentration, comprises:

inputting the first effective chamber concentration/the third effective chamber concentration and the second effective chamber concentration/the fourth effective chamber concentration into a preset synergistic pharmacodynamic model which corresponds to the first medication/the third medication and the second medication/the fourth medication, so as to obtain the current anesthetic effect on the target object;

wherein the preset synergistic pharmacodynamic model comprises a functional relationship between an effect chamber concentration and a probability of not responding to stimulation.

**12.** The method according to any one of claims 1-5, **characterized in that**, further comprising:

receiving the first administration amount/the third administration amount of the first medication/the third medication and the second administration amount/the fourth administration amount of the second medication/the fourth medication, which administration amounts are preset by a user for a preset future time period;

obtaining a trend of change(s) in an anesthetic effect within the preset future time period, based on the first administration amount/the third administration amount and the second administration amount/the fourth administration amount within the preset future time period; and

displaying the trend of change(s) in said anesthetic effect within the preset future time period.

**13.** The method according to claim 12, **characterized in that**, further comprising:

acquiring a target anesthetic effect, which is inputted by the user or is preset;

displaying the target anesthetic effect, when the trend of change(s) in said anesthetic effect within the preset future time period is displayed; and/or

obtaining a time length, which is required for the current anesthetic effect to reach the target anesthetic effect, according to the target anesthetic effect and the trend of change(s) in said anesthetic effect within the preset future time period; and further displaying the time length, when the trend of change(s) in said anesthetic effect within the preset future time period is displayed.

**14.** An apparatus for indicating a depth of anesthesia, **characterized in that**, comprising:

a processor, which is configured to acquire respective administration amounts of at least two medications, which medications are administrated to a target object during an anesthesia process; and configured to obtain, based on the respective administration amounts of the at least two medications, a current anesthetic effect on the target object, wherein the current anesthetic effect is a synergistic effect of the at least two medications on the target object; wherein at least one of the at least two medications is administrated to the target object through an intravenous infusion; and

a human-machine interaction apparatus, which is configured to display the current anesthetic effect;

wherein the current anesthetic effect comprises at least one of: a current equivalent MAC value; equivalent amount information of either of the at least two medications; equivalent amount information of a medication, which medication is not among the at least two medications, but is capable of being administrated to the target object to generate an anesthetic effect; a probability of responding to stimulation; and a probability of not responding to stimulation.

**15.** The apparatus according to claim 14, **characterized in that**, when displaying, through the human-machine interaction apparatus, the current anesthetic effect, the processor is further configured to display through the human-machine interaction apparatus, in a textual or graphic manner, an operation which is suitable for the target object under the current anesthetic effect;

wherein the operation comprises intubation, skin incision, maintaining or awakening.

**16.** The apparatus according to claim 14, **characterized in that**, the processor is further configured to:

estimate metabolism time of anesthetic for the target object according to the current anesthetic effect, and

display the metabolism time of anesthetic, when the current anesthetic effect is displayed.

**17.** The apparatus according to claim 16, **characterized in that**, the metabolism time of anesthetic comprises awakening time after stopping current medication administration;

wherein in order to estimate metabolism time of anesthetic for the target object according to the current anesthetic effect, the processor is further configured to:

acquire an anesthetic effect for awakening, which is inputted by a user or is preset; and

estimate, the metabolism time of anesthetic for the target object, from the current anesthetic effect to the anesthetic effect for awakening, wherein the metabolism time of anesthetic for the target object is the awakening time after stopping current medication administration for the target object.

**18.** The apparatus according to any one of claims 14-17, **characterized in that**, the processor is further configured to:

acquire a safety range of an anesthetic effect, which is inputted by a user or is preset; and
determine whether the current anesthetic effect is within the safety range of said anesthetic effect, and output alarm indication information, if the current anesthetic effect is not within the safety range of said anesthetic effect.

**19.** The apparatus according to any one of claims 14-17, **characterized in that**, the processor is further configured to:

receive an instruction to view a change condition of an anesthetic effect;
generate, in response to said instruction, a trend of change(s) in said anesthetic effect, according to anesthetic effects at different time points during the anesthesia process; and
display, through the human-machine interaction apparatus, the trend of change(s) in said anesthetic effect.

**20.** The apparatus according to claim 19, **characterized in that**, the processor is further configured to:

generate a trend of change(s) in an administration amount of medication, according to administration amounts of medication at different time points during the anesthesia process; and display, in the trend of change(s) in said anesthetic effect, the trend of change(s) in the administration amount of medication; wherein the trend of change(s) in said anesthetic effect and the trend of change(s) in the administration amount of medication share a common coordinate axis of time; or
receive an instruction to select a time point in the trend of change(s) in said anesthetic effect, and display, in response to said instruction, an administration amount of medication which corresponds to the selected time point.

**21.** The apparatus according to claim 14, **characterized in that**, the processor is further configured to:

receive the respective administration amounts of the at least two medications, which administration amounts are preset by a user for a preset future time period;
obtain a trend of change(s) in an anesthetic effect within the preset future time period, based on the respective administration amounts of the at least two medications within the preset future time period; and
display, through the human-machine interaction apparatus, the trend of change(s) in said anesthetic effect within the preset future time period.

**22.** The apparatus according to claim 21, **characterized in that**, the processor is further configured to:

acquire a target anesthetic effect, which is inputted by the user or is preset;
display, through the human-machine interaction apparatus, the target anesthetic effect, when the trend of change(s) in said anesthetic effect within the preset future time period is displayed; and/or
obtain a time length, which is required for the current anesthetic effect to reach the target anesthetic effect, according to the target anesthetic effect and the trend of change(s) in said anesthetic effect within the preset future time period; and further display, through the human-machine interaction apparatus, the time length, when the trend of change(s) in said anesthetic effect within the preset future time period is displayed.

**23.** The apparatus according to claim 21, **characterized in that**, the processor is further configured to:

receive, from the user, a modification to an administration amount of at least one medication of the at least two medications;
update the trend of change(s) in said anesthetic effect within the preset future time period, according to the modification from the user to said administration amount of said at least one medication.

**24.** The apparatus according to any one of claims 14-23, **characterized in that**, the equivalent amount information comprises an equivalent concentration value or an equivalent dosage value.

**25.** An anesthesia machine, **characterized in that**, comprising:

a delivery apparatus for anesthetic, which is configured to deliver to a target object, a gas which is mixed with at least two inhalants;
an anesthesia respiratory apparatus, which is configured to provide a respiratory support to the target object; and
a processor, which is configured to acquire respective administration amounts of the at least two inhalants, which inhalants are administrated to the target object during an anesthesia process; and configured to obtain, based on the respective administration amounts of the at least two inhalants, a current anesthetic effect on the target object, wherein the current anesthetic effect is a synergistic effect of the at least two inhalants on the target object;

wherein the current anesthetic effect comprises at least one of: equivalent amount information of a first inhalant of the at least two inhalants; equivalent amount information of a second inhalant of the at least two inhalants; or equivalent amount information of a medication, which medication is not the first inhalant or the second inhalant, but is capable of being administered to the target object to generate an anesthetic effect.

26. An anesthesia machine, **characterized in that**, comprising:

a delivery apparatus for anesthetic, which is configured to deliver to an anesthesia respiratory apparatus, a gas which is mixed with a medication;

a respiratory loop, which is connected with the anesthesia respiratory apparatus and a target object;

the anesthesia respiratory apparatus, which is configured to provide a respiratory support to the target object, through the respiratory loop;

a monitoring system, which is configured to monitor parameter(s) which is(are) related to ventilation;

a human-machine interaction apparatus, which is configured to display visual information; wherein the visual information comprises a main interface, wherein the main interface comprises a numerical display area which is configured to display value(s) of at least some of the ventilation related parameter(s), and a waveform display area which is configured to display waveform(s) of at least some of the ventilation related parameter(s); and

a processor, which is configured to acquire a third administration amount of a third medication, which medication is administrated to the target object through the respiratory loop during an anesthesia process; configured to acquire a fourth administration amount of at least one fourth medication, which medication is administrated to the target object through an intravenous infusion during the anesthesia process; configured to obtain, based on the third administration amount, a current MAC value of the third medication, which medication individually produce an effect on the target object; and configured to obtain, based on the third administration amount and the fourth administration amount, a current anesthetic effect on the target object; wherein the current anesthetic effect comprises a current equivalent MAC value of the third medication and the fourth medication, which medications synergistically produce effects on the target object, and the current anesthetic effect is configured to indicate a depth of anesthesia of the target object;

wherein the processor is further configured to control the main interface to display, on a same display screen, the current MAC value and the current equivalent MAC value.

27. The anesthesia machine according to claim 26, **characterized in that**, the main interface further comprises an indication area for inhalation anesthesia, which is configured to display parameter(s) which is(are) related to administration of the third medication;

wherein the processor is further configured to display, in the indication area for inhalation anesthesia, the current MAC value; and to display, in an area which is different from the indication area for inhalation anesthesia, the current equivalent MAC value.

28. A computer-readable storage medium on which program(s) is(are) stored, **characterized in that**, the program(s) is(are) capable of being executed by a processor to implement the method according to any one of claims 1-13.

FIG. 1

FIG. 2

Acquiring dosage of medicine — 1

Determining, based on third dosage and fourth dosage, current anesthetic effect on target object, which anesthetic effect is synergistic effect of third medicine and fourth medicine — 2

Displaying current anesthetic effect — 3

FIG. 3

Determines third effect chamber concentration of third medicine based on third dosage, and fourth effect chamber concentration of fourth medicine based on fourth dosage — 21

Obtaining current anesthetic effect on target object, based on third effect chamber concentration and fourth effect chamber concentration — 22

FIG. 4

Medication 1: dosage → Medication 1: pharmacokinetic mode → Medication 1: effect chamber concentration

Medication 2: dosage → Medication 2: pharmacokinetic mode → Medication 2: effect chamber concentration

⋮ ⋮

Medication n: dosage → Medication n: pharmacokinetic mode → Medication n: effect chamber concentration

Pharmacodynamic model → Current Pharmacodynamic effect → Equivalent MAC value

**FIG. 5**

Intravenous infusion

k10

Second chamber ←k12→ ←k21→ Central chamber ←k13→ ←k31→ Third chamber

k1e

Effect chamber

ke0

**FIG. 6**

FIG. 7

FIG. 8

sevo-remif interaction

FIG. 9

FIG. 10

iMAC 1.2

Total Effect

Intubation TOL 90

Skin incision MAC 90

Maintaining MAC 50

Awakening TOSS 50

FIG. 11

## INTERNATIONAL SEARCH REPORT

| International application No. |
| --- |
| **PCT/CN2021/120024** |

**A.      CLASSIFICATION OF SUBJECT MATTER**

A61B 5/00(2006.01)i

According to International Patent Classification (IPC) or to both national classification and IPC

**B.      FIELDS SEARCHED**

Minimum documentation searched (classification system followed by classification symbols)

A61B5/-

Documentation searched other than minimum documentation to the extent that such documents are included in the fields searched

Electronic data base consulted during the international search (name of data base and, where practicable, search terms used)

WPI, EPODOC, CNKI, CNPAT: 深圳迈瑞, 麻醉, 深度, 程度, 指示, 显示, 药, 量, 静脉, 呼吸, 吸入, 两种, 种类, 共同, 协同, 作用, 药效, 等效, 计量, 浓度, 概率, anesthesia, grade, indicat+, drug, dose, vein, breath, inhale, two, kind, display+, cooperate, effect, equivalent, concentration, probability

**C.      DOCUMENTS CONSIDERED TO BE RELEVANT**

| Category* | Citation of document, with indication, where appropriate, of the relevant passages | Relevant to claim No. |
| --- | --- | --- |
| X | US 2009205654 A1 (DRAGER MEDICAL AG. & CO., KG.) 20 August 2009 (2009-08-20) description, paragraphs [0023]-[0036], and figures 1-3 | 1-28 |
| X | CN 103418067 A (DRAGER MEDICAL AG. & CO., KG. et al.) 04 December 2013 (2013-12-04) description, paragraphs [0023]-[0037], and figures 1-5 | 1-28 |
| X | US 2011105914 A1 (DRAGER MEDICAL AG. & CO., KG. et al.) 05 May 2011 (2011-05-05) description, paragraphs [0078]-[0088], and figures 1-4 | 1-28 |
| A | CN 104307069 A (GUANGZHOU GENERAL HOSPITAL OF GUANGZHOU MILITARY COMMAND) 28 January 2015 (2015-01-28) entire document | 1-28 |
| A | CN 103637798 A (QILU HOSPITAL OF SHANDONG UNIVERSITY) 19 March 2014 (2014-03-19) entire document | 1-28 |

☑ Further documents are listed in the continuation of Box C.          ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **29 November 2021** | **22 December 2021** |

| Name and mailing address of the ISA/CN | Authorized officer |
| --- | --- |
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/120024**

| C. | DOCUMENTS CONSIDERED TO BE RELEVANT | |
|---|---|---|
| Category* | Citation of document, with indication, where appropriate, of the relevant passages | Relevant to claim No. |
| A | CN 101745166 A (SHENZHEN MINDRAY BIO-MEDICAL ELECTRONICS CO., LTD.) 23 June 2010 (2010-06-23)<br>    entire document | 1-28 |
| A | CN 102355913 A (HOPITAL FOCH) 15 February 2012 (2012-02-15)<br>    entire document | 1-28 |

Form PCT/ISA/210 (second sheet) (January 2015)

**INTERNATIONAL SEARCH REPORT**
**Information on patent family members**

International application No.

**PCT/CN2021/120024**

| Patent document cited in search report | | | Publication date (day/month/year) | Patent family member(s) | | | Publication date (day/month/year) |
|---|---|---|---|---|---|---|---|
| US | 2009205654 | A1 | 20 August 2009 | US | 2006081244 | A1 | 20 April 2006 |
| | | | | US | 7556036 | B2 | 07 July 2009 |
| | | | | DE | 102004050717 | B3 | 29 September 2005 |
| | | | | US | 8662077 | B2 | 04 March 2014 |
| | | | | FR | 2876590 | A1 | 21 April 2006 |
| | | | | FR | 2876590 | B1 | 04 June 2010 |
| CN | 103418067 | A | 04 December 2013 | DE | 102012009617 | A1 | 21 November 2013 |
| | | | | DE | 102012009617 | B4 | 26 March 2020 |
| | | | | US | 2013306063 | A1 | 21 November 2013 |
| | | | | US | 9744299 | B2 | 29 August 2017 |
| | | | | CN | 103418067 | B | 02 March 2016 |
| | | | | FR | 2990617 | A1 | 22 November 2013 |
| | | | | FR | 2990617 | B1 | 06 October 2017 |
| | | | | JP | 3185202 | U | 08 August 2013 |
| US | 2011105914 | A1 | 05 May 2011 | US | 10285597 | B2 | 14 May 2019 |
| | | | | DE | 102007038975 | A1 | 02 January 2009 |
| | | | | US | 2009005655 | A1 | 01 January 2009 |
| | | | | US | 7878982 | B2 | 01 February 2011 |
| | | | | FR | 2917980 | A1 | 02 January 2009 |
| | | | | FR | 2917980 | B1 | 05 July 2013 |
| CN | 104307069 | A | 28 January 2015 | CN | 104307069 | B | 31 May 2017 |
| CN | 103637798 | A | 19 March 2014 | CN | 103637798 | B | 09 March 2016 |
| CN | 101745166 | A | 23 June 2010 | CN | 101745166 | B | 11 April 2012 |
| CN | 102355913 | A | 15 February 2012 | WO | 2010081947 | A1 | 22 July 2010 |
| | | | | JP | 2012515032 | A | 05 July 2012 |
| | | | | JP | 5792629 | B2 | 14 October 2015 |
| | | | | FR | 2940912 | A1 | 16 July 2010 |
| | | | | FR | 2940912 | B1 | 16 August 2013 |
| | | | | EP | 2389208 | A1 | 30 November 2011 |
| | | | | EP | 2389208 | B1 | 29 April 2020 |
| | | | | US | 2012010591 | A1 | 12 January 2012 |
| | | | | US | 10537677 | B2 | 21 January 2020 |
| | | | | CN | 102355913 | B | 31 December 2014 |

Form PCT/ISA/210 (patent family annex) (January 2015)